Europäisches Patentamt

European Patent Office

Office européen des brevets

(11) Publication number: **0 462 836 A2**

# EUROPEAN PATENT APPLICATION

(21) Application number: 91305596.8

(22) Date of filing: 20.06.91

(51) Int. Cl.⁵: **C12N 15/53, C12N 1/21, C12P 21/02, C12N 9/02, // (C12N1/21, C12R1:19)**

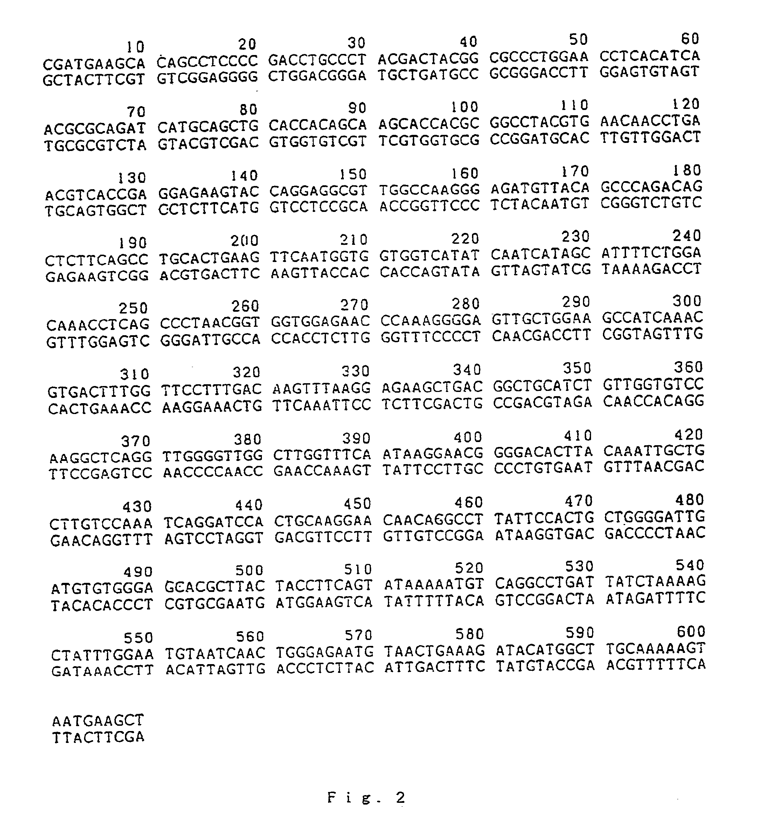

A request for correction of figure 2 has been filed pursuant to Rule 88 EPC. A decision on the request will be taken during the proceedings before the Examining Division (Guidelines for Examination in the EPO, A-V, 2.2).

(30) Priority: 20.06.90 JP 159925/90
19.10.90 JP 279286/90

(43) Date of publication of application:
27.12.91 Bulletin 91/52

(84) Designated Contracting States:
DE FR GB

(71) Applicant: MITSUI TOATSU CHEMICALS, Inc.
2-5 Kasumigaseki 3-chome
Chiyoda-Ku Tokyo 100 (JP)

(72) Inventor: Takahashi, Seishi
245-18, Tsunashima
Mobara-shi, Chiba 297 (JP)
Inventor: Makino, Tadashi
5-14-8, Hiyoshidai
Togane-shi, Chiba 283 (JP)
Inventor: Asanagi, Mineko
2785-1, Mutsuno
Mobara-shi, Chiba 297 (JP)
Inventor: Yoshino, Chie
2785-1, Mutsuno
Mobara-shi, Chiba 297 (JP)

(74) Representative: Harvey, David Gareth et al
Graham Watt & Co. Riverhead
Sevenoaks Kent TN13 2BN (GB)

(54) Recombinant vector plasmid capable of expressing human manganese superoxide dismutase, and process of producing this enzyme.

(57) A recombinant vector for producing human manganese superoxide dismutase (Mn-SOD) and a process of producing human Mn-SOD using the recombinant vector are disclosed. The recombinant vector comprises a ligated promoter including tac promoter and $P_L$ promoter; a structural gene encoding human manganese superoxide dismutase, which is located downstream the ligated promoter; and a terminater which acts in E. coli cell, which is located downstream the structural gene.

EP 0 462 836 A2

BACKGROUND OF THE INVENTION

I. Field of the Invention

This invention relates to a recombinant vector plasmid capable of effectively expressing human manganese superoxide dismutase (hereinafter referred to as "human Mn-SOD"), and to a process of producing human Mn-SOD using the same. The present invention also relates to a process of recovering human Mn-SOD from a solution containing proteins in bacterial cells, including human Mn-SOD, by which a human Mn-SOD same as the naturally occurring human Mn-SOD can be effectively isolated.

II. Description of the Related Art

Superoxide dismutase (hereinafter referred to as "SOD") is an enzyme widely occurring in most organisms as an essential enzyme. This enzyme controls anti-oxidation properties and removes active oxygen produced by phagocytosis of luekocytes or the like so as to protect the organism from the active oxygen. Most of the active oxygen with high reactivity is usually removed by SOD. However, recent studies revealed that excess active oxygen cannot be removed at once and the thus accumulated active oxygen destroys the cells and tissues so as to cause inflammation. Such inflammation may occur at any part of the body and may cause critical results. Recent clinical studies of such inflammation revealed that the inflammation is caused by free radical. Thus, the role of SOD in organisms is very important. In these several years, the properties of various SODs have been compared. As a result, it was discovered that Mn-SOD plays more important role than other SODs and that Mn-SOD is very stable in the body (Reference 11). Thus, it is desired to produce pure human Mn-SOD in a large scale by using genetic engineering technique.

A part of the amino acid sequence of human Mn-SOD was determined by Harris et al in 1977 (Reference 1) and the whole amino acid sequence was determined by Barra et al in 1984 (Reference 2). The SOD studied by Barra et al was isolated from human liver. The study revealed that Mn-SOD has a novel amino acid sequence which is utterly different from those of Cu-SOD and Zn-SOD. Recently, cDNAs of Mn-SOD were cloned from human cells of different origins and Mn-SOD genes encoding amino acid sequences in which several amino acids are different each other were identified (References 3, 4 and 5). The determined amino acid sequences are similar to that determined by Barra et al (Reference 2).

These human Mn-SODs have been produced by genetic engineering technique using E. coli (References 3, 13 and 18) or using yeasts (Reference 2).

In the method of producing human Mn-SOD using E. coli, E. coli transformed with a vector containing human Mn-SOD gene located at downstream region of $P_L$ promoter is used. $P_L$ promoter is a known promoter originated from λ phage. However, in the metod in which $P_L$ promoter is used, to produce the desired protein, the culturing temperature must be changed in the later stage of the culturing so as to induce the production of the desired protein. Although such a method may be effective in some cases where the desired protein is toxic to the host cell, in cases where the desired protein is not toxic to the host cell as in the case of producing human Mn-SOD, changing the culturing temperature is merely troublesome, and moreover, it may result in the formation of incomplete molecules due to the rapid protein synthesis. Further, the percentage of the desired human Mn-SOD accumulated in the host cells among the soluble proteins in the host cells is only 20% (References 13 and 18). Still further, the human Mn-SOD produced by this process has a manganese ion content of 0.77 ion per one polypeptide chain, which is insufficient for human Mn-SOD to exhibit the SOD activity. The human Mn-SOD extracted from human organs is tetramer (Reference 17). With the conventional method for producing human Mn-SOD using E. coli., the content of tetramer in the produced human Mn-SOD is only 10% (Reference 3).

In the conventional process in which a yeast is used, the amount of the produced desired protein is only 0.5 mg/l medium (Reference 14), which is much smaller than that (400 - 500 mg/l) attained by the process of the present invention later described. Further, culturing yeasts is more troublesome than culturing E. coli.

For the effective production of a polypeptide having human Mn-SOD enzyme activity, it is advantageous in various respects to use E. coli. However, with the conventional technique, as mentioned above, satisfactory production process of human Mn-SOD has not yet been established.

SUMMARY OF THE INVENTION

Accordingly, an object of the present invention is to provide a recombinant vector plasmid capable of effectively producing human Mn-SOD in a large amount in E. coli.

Another object of the present invention is to provide a process of producing human Mn-SOD using the recombinant vector plasmid of the present invention, by which human Mn-SOD with high manganese content and high activity may be effectively produced in a large amount by a simple operation.

Still another object of the present invention is to provide a process of recovering human Mn-SOD from a solution containing soluble proteins in the host cells, by which the molecular weight and specific activity of the human Mn-SOD are not decreased and human Mn-SOD having the same molecular weight and high activity as those of naturally occurring human Mn-SOD may be isolated.

The present invention provides a recombinant vector comprising a ligated promoter including tac promoter and P$_L$ promoter; a structural gene encoding human Mn-SOD, which is located downstream the ligated promoter; and a terminator which acts in E. coli cell, which is located downstream the structural gene.

The present invention also provides an E. coli transformant which is transformed with the recombinant vector plasmid of the present invention.

The present invention further provides a process of producing human Mn-SOD comprising culturing the transformant of the present invention in a condition to produce the human Mn-SOD, and recovering the produced human Mn-SOD.

The present invention still further provides a process of recovering human Mn-SOD from a solution containing soluble proteins in bacterial cells, comprising, in the order mentioned, the steps of: (a) passing the solution containing soluble proteins in the bacterial cells through an anion-exchange chromatography column; (b) adjusting the pH of the passed through fluid from the anion-exchange chromatography column to 6.0 - 6.7, and adjusting the salt concentration of the passed through fluid from the anion-exchange chromatography column to a degree capable of being adsorbed to a cation-exchange chromatography column used in the following step (c); (c) passing the resulting solution obtained in step (b) through a cation-exchange chromatography column; (d) passing through the cation-exchange chromatography column an elution buffer having a pH of 6.0 - 6.7 and a high salt concentration capable of eluting human Mn-SOD; and (e) recovering the eluted human Mn-SOD.

By the present invention, it was first attained to produce human Mn-SOD in a large amount effectively by means of genetic engineering technique. In the method of producing human Mn-SOD according to the present invention, the troublesome induction operation such as raising the culturing temperature or the like is not necessary, so that the process is simple. Further, the human Mn-SOD recovered by the process of the present invention is mostly in the form of tetramer, so that the human Mn-SOD retains its high activity.

The invention will now be described in more detail by way of example only, with reference to the accompanying drawings in which:

Fig. 1 shows amino acid sequence of human Mn-SOD;

Fig. 2 shows nucleotide sequence encoding the polypeptide shown in Fig. 1;

Fig. 3 shows nucleotide sequence shown in Fig. 2 divided into three blocks as well as DNA fragments further divided for the purpose of chemical synthesis;

Fig. 4 shows procedures for preparing a recombinant vector plasmid pSWTPSOD;

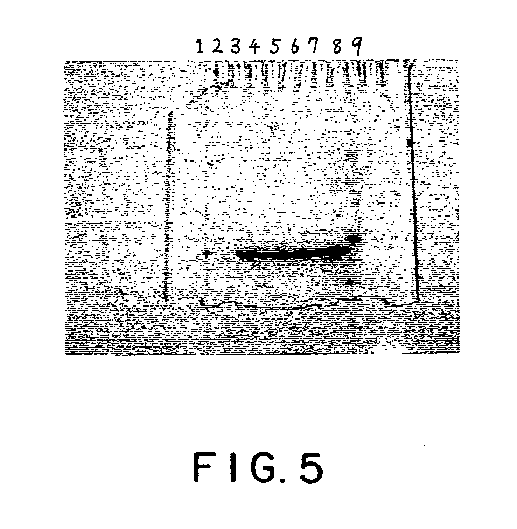

Fig. 5 shows results of SDS electrophoresis (CBB staining) of purified human Mn-SOD protein, wherein Lane 1 shows the pattern of the supernatant of disrupted cell suspension, Lane 2 shows the pattern of the fluid after DEAE-Sepharose column chromatography, Lane 3 shows the pattern of the concentrated fluid after DEAE-Sepharose column chromatography, Lane 4 shows the pattern of the fluid after the buffer exchange using Sephadex G-25, Lane 5 shows the pattern of the fluid after CM-Sepharose column chromatography, Lane 6 shows the pattern of Mn-SOD marker originated from E. coli, Lane 7 shows the pattern of Mn-SOD marker originated from E. coli, Lane 8 shows the pattern of human Mn-SOD marker originated from human liver, and Lane 9 shows the pattern of molecular weight markers;

Fig. 6 shows procedures for preparing plasmid pP$_L$SOD;

Fig. 7 shows amino acid sequence of modified human Mn-SOD in which 59th isoleucine is converted to threonine;

Fig. 8 shows nucleotide sequence encoding the polypeptide shown in Fig. 9; and

Fig. 9 is a restriction map of plasmid pSWTPTSOD.

## DETAILED DESCRIPTION OF THE PREFERRED EMBODIMENTS

As described above, the recombinant vector plasmid of the present invention contains a ligated promoter including tac promoter and P$_L$ promoter. The tac promoter is a fused promoter having the E. coli tryptophan promoter and E. coli lactose promoter, which per se is well-known in the art. The P$_L$ promoter is originated from E. coli λ phage, which per se is also well-known in the art.

In general, although various excellent promoters are known, since the suitability of a promoter with respect to a structural gene downstream the promoter varies depending on the properties of the structural gene or the polypeptide encoded thereby, such as cytotoxicity of the polypeptide, it is impossible to determine the suitability of a promoter when a specific structural gene is to be expressed. Thus, although various promoters which function in E. coli such as lac promoter, tac promoter and $P_L$ promoter are known, for the production of human Mn-SOD, only $P_L$ promoter alone has been employed (Reference 3). However, as mentioned above, if $P_L$ promoter alone is employed, a troublesome operation to change the culturing temperature when the protein is expressed is necessary and the amount of the produced desired protein is not sufficiently. In contrast, in the vector plasmid of the present invention containing a ligated promoter having tac promoter and $P_L$ promoter is used, the induction of production by raising the culturing temperature is not necessary, and the amount of the produced human Mn-SOD is larger.

In the conventional process, $P_L$ promoter which can be induced by raising temperature was employed (References 3 and 13) in order to eliminate the possible damage by hydrogen peroxide which is the intrinsic reaction product formed by SOD. It was surprisingly discovered by the present inventors that if tac/$P_L$ ligated promoter is employed, preferably with the addition of manganese ion to the culturing medium, human Mn-SOD is continuously produced and a large amount of (twice the amount attained when $P_L$ promoter is employed) human Mn-SOD is accumulated keeping its activity. It was also discovered that the human Mn-SOD accumulated in a large amount in the host cells does not inhibit the growth of the host cells and the production of the desired protein, so that the production of the enzyme in a large amount may be accomplished.

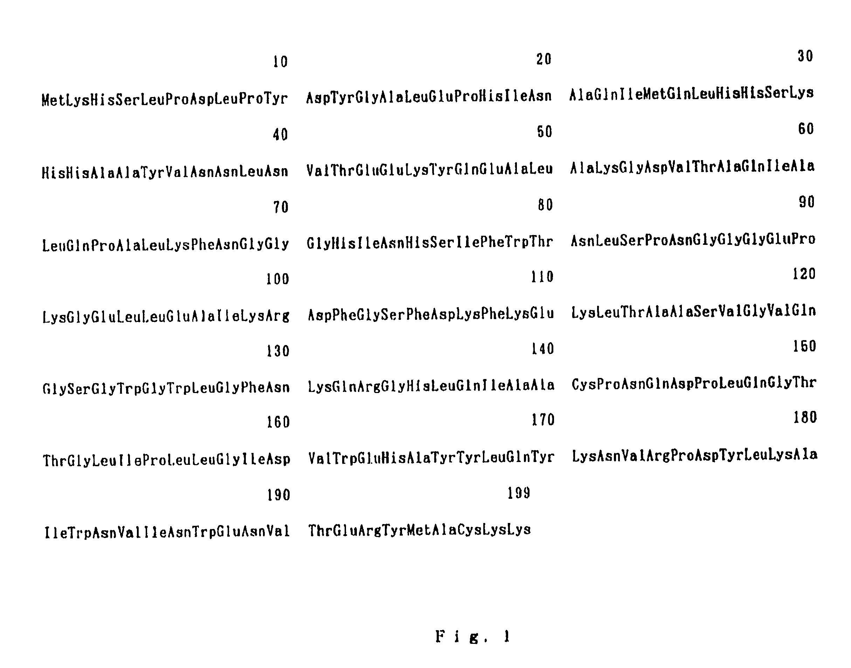

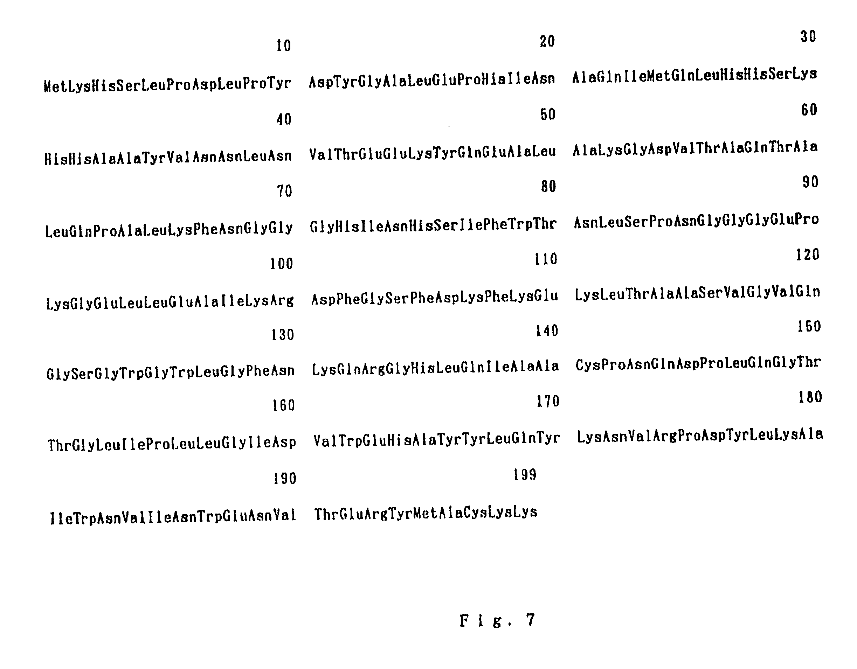

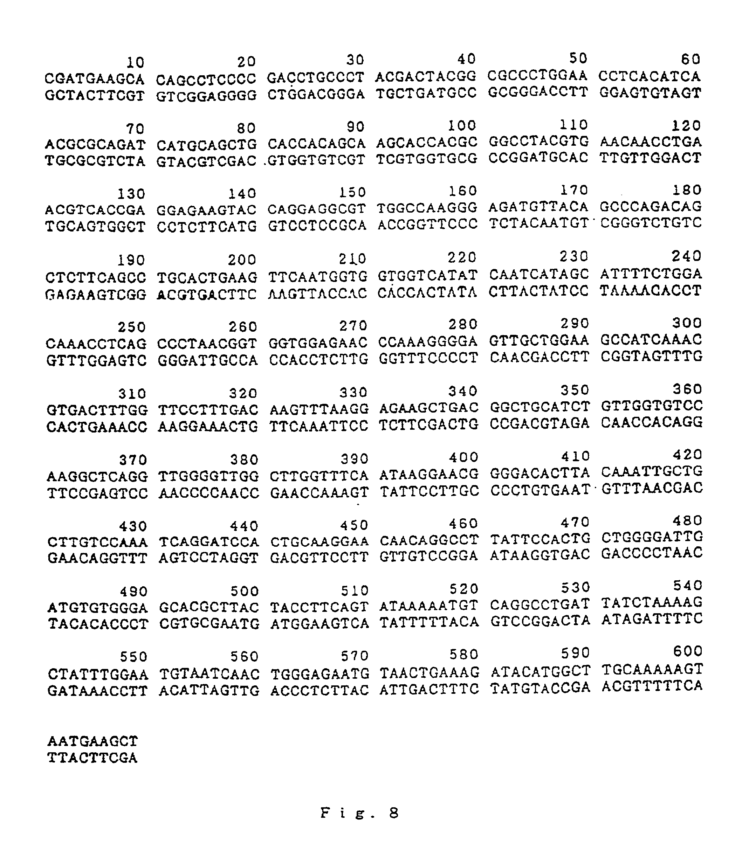

In the recombinant vector plasmid of the present invention, at downstream region of the above-mentioned promoter, a structural gene encoding human Mn-SOD is operatively linked with the promoter. The human Mn-SOD gene per se is known, which encodes the amino acid sequence, for example, shown in fig. 1 or 7. Examples of the nucleotide sequence of the human Mn-SOD gene are shown in Figs. 2 and 8. The human Mn-SOD gene may be isolated by cloning the gene from cDNAs prepared from an appropriate human tissue as described in the published references (References 3, 4 and 5). Alternatively, the human Mn-SOD gene may be divided into some blocks and each of the blocks is chemically synthesized, followed by ligation of the blocks so as to form the complete gene, which is employed in the actual working examples hereinbelow described.

Downstream the structural gene of human Mn-SOD, a terminator is operatively linked. Any terminator may be employed as long as it functions in E. coli. The rrnB terminator for ribosomal RNA translation is especially preferred.

The recombinant vector plasmid containing tac/$P_L$ ligated promoter may be constructed by firstly inserting $P_L$ promoter fragment by a known recombination technique in a plasmid such as pKK223-3 containing tac promoter and a terminator at downstream region of the tac promoter, and then inserting the human Mn-SOD gene immediately downstream the $P_L$ promoter.

The expression unit including the tac/$P_L$ ligated promoter, the human Mn-SOD structural gene and the terminator may be cloned in a plasmid. Any plasmid such as pBR322, pUC18 or pKK223-3 which can transform E. coli may be employed.

The above-mentioned expression unit may be inserted in any region of the plasmid as long as it can be expressed. In cases where the expression unit is inserted in a plasmid of pBR series or pUC series having ampicillin-resistant gene, the expression unit may preferably be inserted in, for example, Eco RI - Hind III site or Eco RI - Bam HI site.

As the host, transformable E. coli usually used in the field of genetic engineering may be employed. Specific examples of the E. coli strains which may be employed include E. coli C600, HB101, MC1061, DH5 α, JM105, JM109 and the like. Among these, E. coli JM105 and JM109 are preferred. Since the host E. coli usually produces its own Mn-SOD, the bacterial Mn-SOD may be contaminated in the desired human Mn-SOD, so that the bacterial Mn-SOD must be removed during the purification step of the human Mn-SOD. Thus, if an E. coli host which does not produce its Mn-SOD is employed, it is very advantageous for the industrial production of human Mn-SOD. An example of such E. coli which does not produce Mn-SOD is E. coli GC4468 (Reference 19).

E. coli host may be transformed by a conventional method. For example, E. coli is grown in a nutrient medium until the absorbance at 600 nm reaches 0.4 - 0.6. After collecting the cells, the cells are treated with a buffer containing rubidium chloride or calcium chloride and the recombinant vector plasmid, thereby introducing the vector plasmid into the host cells (Reference 7). The selection of the transformant may be carried out by a conventional method in which the transformant is selected according to the selection marker such as drug resistance of the plasmid and the human Mn-SOD-producing cells are then selected.

The human Mn-SOD may be obtained by culturing the transformant which produces human Mn-SOD and recovering the Mn-SOD from the cells. The cells may be cultured at a temperature of 32 - 40°C. A usual nutrient medium for culturing E. coli (e.g., those described in Reference 7) to which manganese ion is added, may be employed. The concentration of manganese ion may be 1 - 20 mM, preferably 10 - 15 mM. After culturing the host cells, the cells are collected by centrifugation. The collected cells are then disrupted by a conventional method such as changing the pressure, treatment with glass beads or with lysozyme. The insoluble materials are removed by centrifugation.

From the resulting supernatant which is a solution containing soluble proteins in the host cells, the desired human Mn-SOD may be purified by anion-exchange chromatography and then cation-exchange chromatography in this order appropriately adjusting the pH and the salt concentration of the buffer. This method comprises the above-described steps (a) to (e).

More particularly, the solution or the supernatant containing the soluble proteins in the host cells obtained in the preceding step is firstly passed through an anion-exchange chromatography column. As the anion-exchanger employed in the chromatography, those having amino groups are preferred. Among these, those having tertiary amino groups, and those having quaternary ammonium salt are preferred. Preferred examples of the anion-exchanger used herein include DEAE-Sepharose, DEAE-Sephacel and Q-Sepharose (all of them are commercially available from Pharmacia). As the buffer for adsorption and washing, phosphate buffer and tris buffer may be employed. The buffer may preferably have a pH of about 7.8 and a salt concentration of 10 - 30 mM. By this anion-exchange chromatography, many contaminants may be removed.

In the next step, the pH and the salt concentration of the passed through fluid from the preceding anion-exchange chromatography are adjusted. The pH of the passed through fluid is adjusted to 6.0 - 6.7, preferably about 6.5. The salt concentration of the passed through fluid is adjusted to a degree which allows the adsorption of the protein in the following cation-exchange chromatography step (c). More particularly, in the case where the pH of the passed through fluid is about 6.5, a salt concentration of 2 - 10 mM is preferred. The pH and the salt concentration of the passed through fluid from the anion-exchange chromatography may be adjusted by conventional methods such as gel permeation chromatography, dialysis and the like.

The thus adjusted passed through fluid from the anion-exchange chromatography is then applied to a cation-exchange chromatography column. As the cation-exchanger, those having carboxymethyl groups or sulfone groups are preferably employed. Preferred examples of the cation-exchanger which may be employed include CM-Sepharose and S-Sepharose (both are commercially available from Pharmacia). By this cation-exchange chromatography, pure human Mn-SOD may be isolated. The buffer used for adsorption and washing has the pH and salt concentration as mentioned above for the conditioned passed through fluid from the anion-exchange chromatography. The buffer used for elution has a pH of 6.0 - 6.7, preferably about 6.5, and a high salt concentration capable of eluting human Mn-SOD. The salt concentration may preferably be 20 - 40 mM, especially about 30 mM, in the case where the buffer used for elution has a pH of about 6.5.

Among the eluted fractions, those exhibiting SOD activity are recovered.

It was surprisingly discovered that by employing this process, the human Mn-SOD in the form of tetramer which is the active form of this enzyme may be obtained with a high yield. Further, the human Mn-SOD purified by this method has a high manganese ion content of not less than 0.9 ion per one polypeptide chain, so that it has a specific activity as high as not less than 4200 units/mg protein. The human Mn-SOD purified by this method has a molecular weight of not less than 85,000. Thus, the human Mn-SOD purified by this method is useful in clinical applications such as anti-inflammation agent.

The present invention will now be described by way of examples thereof. It should be noted that the examples are presented for the illustration purpose only and should not be interpreted in any restrictive way.

In the following examples, unless otherwise specified, each procedure was carried out in accordance with representative conventional method described in Reference 7.

Example 1

Construction of Expression Vector having tac/$P_L$ Promoter

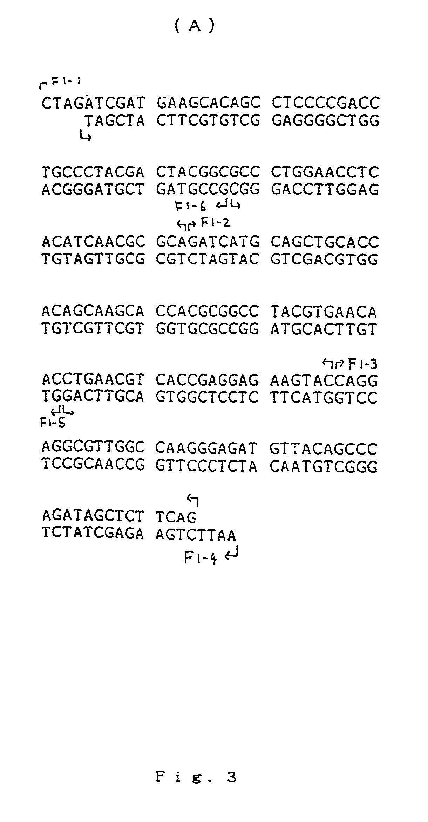

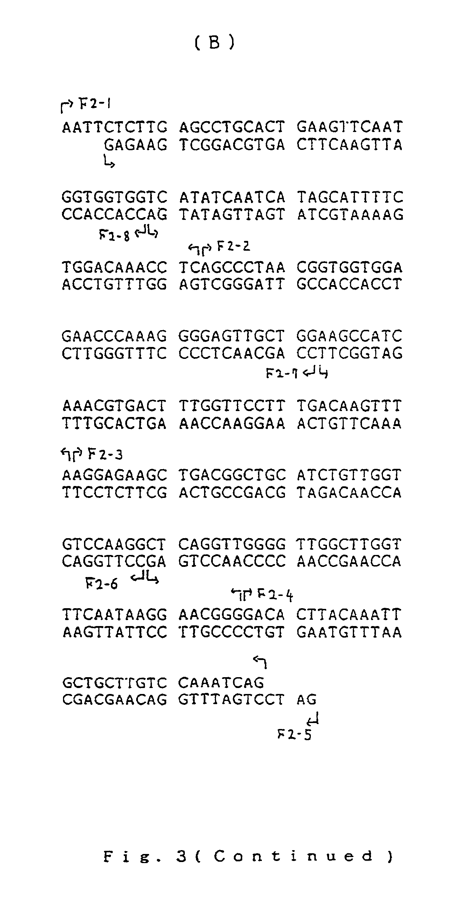

The DNA shown in Fig. 2 encoding the human Mn-SOD having the amino acid sequence shown in Fig. 1 was divided into three blocks A, B and C as shown in Fig. 3, each of the blocks being provided with restriction sites at the ends thereof. Each block is further divided into 6 - 8 sections and these sections were chemically synthesized. The synthesis of the DNA fragments was carried out by the phosphoamidite method (Reference 8) using Model 381A automatic DNA synthesizer commercially available from Applied Biosystems.

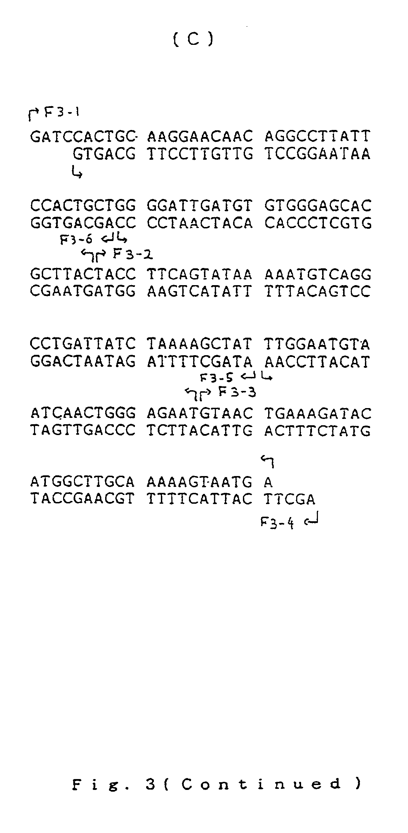

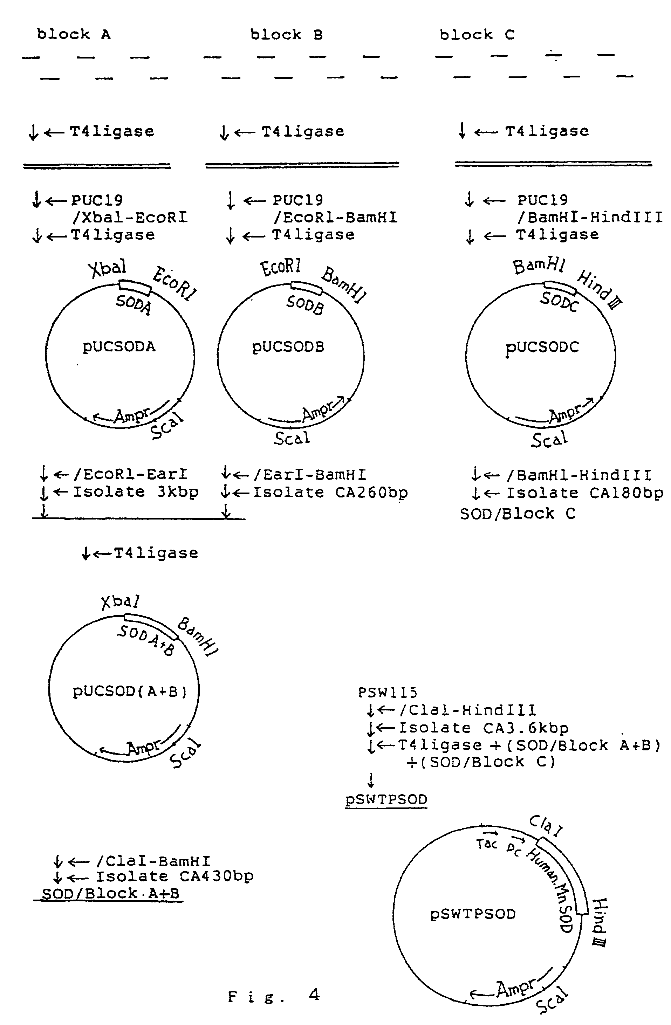

To each chemically synthesized DNA fragment in the amount of 0.2 μg, phosphate group was introduced to the 5' end thereof. The chemically synthesized 6 - 8 DNA fragments constituting the blocks A, B or C shown in fig. 3 were ligated by using T4 ligase so as to form the blocks A, B and C. The block A was digested with Xba I - Eco RI, the block B was digested with Eco RI - Bam HI and the block C was digested with Bam HI - Hind III. By electrophoresis, the desired DNA blocks A, B and C were isolated (Fig. 4).

The DNA blocks A, B and C were inserted in Xba I - Eco RI site, Eco RI - Bam HI site and Bam HI - Hind III site, respectively, of pUC plasmid vector using T4 DNA ligase and cloned so as to obtain plasmids pUCSODA (containing block A), pUCSODB (containing block B), pUCSODC (containing block C).

The three plasmids were amplified in E. coli and purified. The plasmid pUCSODA was digested with Eco RI - Ear I and a DNA fragment of about 3 kbp was recovered. The plasmid pUCSODB was digested with Ear I - Bam HI and a DNA fragment of about 260 bp was recovered. These two DNA fragments were ligated using T4 DNA ligase to obtain a plasmid pUCSOD(A+B). The plasmid pUCSOD(A+B) was amplified in E. coli and purified. The resulting plasmid pUCSOD(A+B) was digested with Cla I - Bam HI and the resulting SOD fragment (A+B) of about 430 bp was recovered. Plasmid pUCSODC was also amplified in E. coli and was digested with Bam HI - Hind III, followed by recovery of SOD fragment (C) of about 180 bp.

The SOD fragments (A+B) and (C) were inserted in a known plasmid expression vector, plasmid pSW115 (Reference 6) having tac/P_L ligated promoter. The SOD fragments were operatively inserted at downstream region of the ligated promoter. That is, the plasmid pSW115 was digested with Cla I and Hind III and the resulting DNA fragment of about 5.1 kb (hereinafter "Cla I - Hind III fragment") was recovered. The resulting Cla I - Hind III fragment and the two SOD fragments (A+B) and (C) were ligated by using T4 ligase so as to obtain an expression vector pSWTPSOD.

In the above-mentioned procedures, E. coli DH5 α strain (commercially available from Bethesda Research Laboratories, Maryland, U.S.A.) was used.

Example 2

Expression of Human Mn-SOD Using Expression Vector Having tac/P_L Ligated Promoter

E. coli JM109 (commercially available from Toyobo, Osaka, Japan) was transformed with the plasmid pSWTPSOD obtained in Example 1 and a transformat JM109SOD which acquired resistance to ampicillin was selected. The transformant was cultured under shaking in one liter of 2 x LB medium (Reference 7) containing 50 mg/l of ampicillin and 10 mM of manganese sulfate at 37°C for 20 hours. Upon reaching an absorbance at 600 nm to 12.4, the cells were collected by centrifugation and were suspended in a 10 mM potassium phosphate buffer having a pH of 7.8. The cells were then disrupted by glass beads by using Dynomill. The disrupted cell suspension was centrifuged and the supernatant was recovered. the Mn-SOD activity of the supernatant was measured by a conventional Method (Reference 9). The total activity was $2.26 \times 10^6$ units as shown in Table 1. The percentage of the desired human Mn-SOD among the soluble proteins in the supernatant was 45.1% by weight.

Example 3

Isolation of Human Mn-SOD from Supernatant of Disrupted Cell Suspension Prepared in Example 2

The supernatant of the disrupted cell suspension prepared from one liter of the culturing medium in Example 2 was applied to DEAE-Sepharose column (commercially available from Pharmcia) preliminarily equilibrated with 10 mM potassium phosphate buffer (pH 7.8) with a volume of 5 times that of the column. After concentrating the passed through fluid by ultrafiltration, the resulting concentrate was applied to Sephadex G-25 (commercially available from Pharmacia) column and the buffer was changed to 10 mM potassium phosphate buffer (pH 6.5) thereby. The resultant was applied to CM-Sepharose (commercially available from Pharmacia) preliminarily equilibrated with 10 mM potassium phosphate buffer (pH 6.5) with a volume of 5 times that of the column. After washing the column with the same buffer with a volume of 3 times that of the column, the proteins were eluted by 30 mM potassium phosphate buffer (pH 6.5) and fractions exhibiting SOD activity were collected and combined. The thus obtained Mn-SOD solution was subjected to electrophoresis. As a result, as shown in Fig. 5, a single band was obtained, so that it was proved that the protein was isolated. The Mn-SOD solution had a total SOD activity of $7 \times 10^5$ units. The specific activity of the Mn-SOD solution determined in accordance with References 9 and 12 was 5280 units/mg protein.

The manganese content of the thus purified human Mn-SOD, which was measured by atomic absorption spectrophotometry, was 0.99 manganese ion per one polypeptide chain. The molecular weight of the thus isolated protein was determined by low angle laser scattering method (Reference 16) using molecular weight markers of BSA (bovine serum albumin, M.W. 67,000) and OVA (ovalubmin, M.W. 43,000). The determined molecular weight was 98,000, so that it was shown that this human Mn-SOD is in the form of tetramer.

The results of the polyacrylamide-SDS electrophoresis during and after the purification are shown in Fig. 5.

Table 1

| Sample | Amount of Total Protein[1] (g/l medium) | Total SOD Activity[2] (units/l medium) |
|---|---|---|
| Disrupted Cell Suspension | 1755 | $2.26 \times 10^6$ |
| After DEAE- Sepharose Column | 432 | $1.82 \times 10^6$ |
| After CM- Sepharose Column | 146 | $7.7 \times 10^5$ |

[1]: Determined by Lowry method (Reference 12) using BSA as a standard protein

[2]: Determined by Cytochrome C method (Reference 9) by McCord

Example 4

Construction of Expression Vector Containing tac/$P_L$ Ligated Promoter, Which Has Human Mn-SOD Structural Gene Encoding Amino Acid Sequence Shown in Fig. 7.

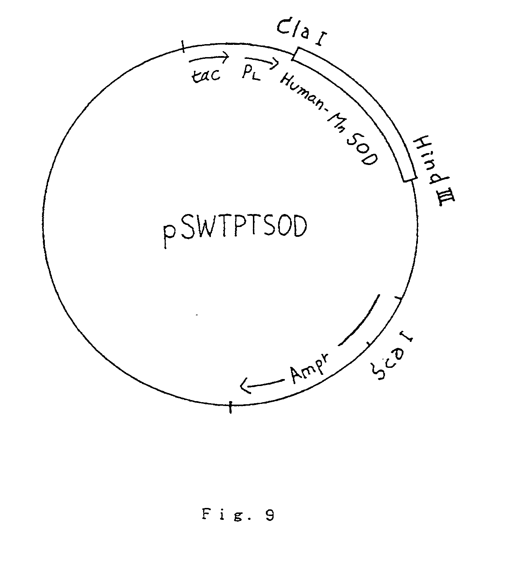

In the same manner as in Example 1, the gene shown in fig. 8 which encodes amino acid sequence of modified human Mn-SOD in which 59th isoleucine is converted to threonine, shown in Fig. 7. More particularly, the gene was divided in the same manner as shown in Fig. 3, and according to the scheme shown in Fig. 4, an expression vector pSWTPTSOD (shown in Fig. 9) expressing human Mn-SOD having the amino acid sequence shown in Fig. 7 was prepared. The human Mn-SOD was expressed according to the same method as in Example 2, and the human Mn-SOD was purified by the same manner as in Example 3. The supernatant of the disrupted cell suspension had a total SOD activity of $1.44 \times 10^6$ as shown in Table 2. The desired human Mn-SOD was obtained as a pure protein which exhibited an activity of $7.9 \times 10^5$ units.

Table 2

| Sample | Amount of Total Protein (g/l medium) | Total SOD Activity (units/l medium) |
|---|---|---|
| Disrupted Cell Suspension | 2981 | $1.44 \times 10^6$ |
| After DEAE-Sepharose Column | 1018 | $9.6 \times 10^6$ |
| After CM-Sepharose Column | 472 | $7.9 \times 10^5$ |

Comparative Example 1

Construction of Plasmid pP$_L$MNSOD and expression of Human Mn-SOD Thereby

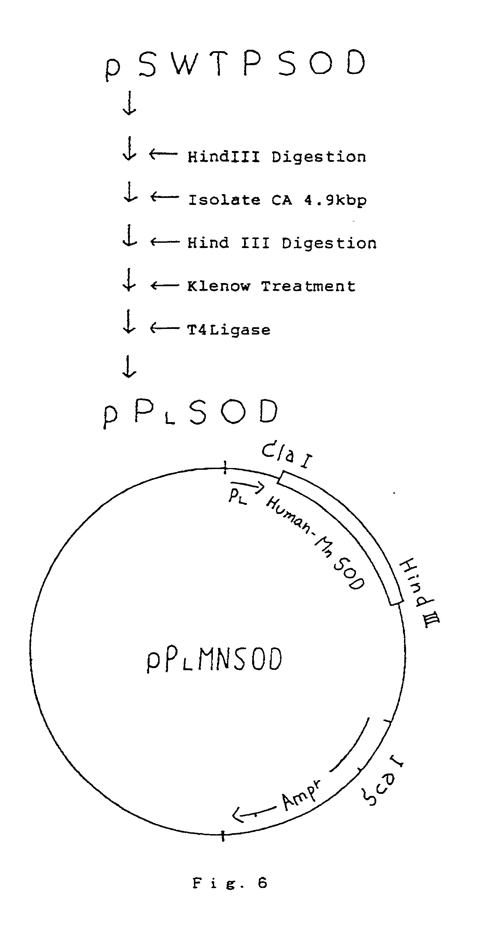

A plasmid pP$_L$MNSOD having P$_L$ promoter alone and human Mn-SOD structural gene was prepared in accordance with the scheme shown in Fig. 6. More particularly, 10 μg of the plasmid pSWTPSOD prepared in Example 1 was digested with Eco RI and the digest was purified by agarose gel electrophoresis to obtain 1 μg of linear plasmid generated by cutting one Eco RI site in the plasmid pSWTPSOD. The linear plasmid was digested with Bam HI to cut the unique Bam HI site therein and DNA fragment of 4.9 kbp was purified by agarose gel electrophoresis. The isolated fragment in the amount of 0.2 μg was treated with DNA polymerase Klenow fragment so as to make its both ends blunt. The thus obtained DNA fragment was circulated by a conventional reaction using T4 ligase and the resulting plasmid was introduced in E. coli DH5 α strain to transform the same. By screening the transformants, 2 transformants among 36 transformants contained the desired plasmid pP$_L$MNSOD which does not have tac promoter.

With the plasmid pP$_L$MNSOD, E. coli N4830 (having cl857 and N gene, commercially available from Pharmacia) was transformed and the transformant was cultured in 1 x LB medium (Reference 7) containing 50 mg/l of ampicillin at 32°C until the absorbance at 600 nm of the culture medium reaches 0.7. To the culture medium, manganese sulfate was added to a final concentration of 1.2 mM and the culture medium was incubated at 42°C for 2 hours. The cells were collected by centrifugation and the collected cells were suspended in 250 mM NaCl buffer containing 50 mM potassium phosphate, followed by disruption of the cells by the treatment with glass beads by using Dynomill. The disrupted cell suspension was centrifuged and the supernatant was recovered. The total Mn-SOD activity of the supernatant was measured, which was $9.84 \times 10^5$ units as shown in Table 3. The percentage of the desired human Mn-SOD among the soluble proteins in the supernatant was 20.1% by weight.

Comparative Example 2

Isolation of Human Mn-SOD from Supernatant of Disrupted Cell Suspension Obtained in Comparative Example 1

The supernatant of the disrupted cell suspension obtained in Comparative Example 1 from one liter of the culture medium was heated at 60°C for one hour and the cooled to 4°C. The resultant was centrifuged and the supernatant was recovered. After concentrating the resulting supernatant by ultrafiltration, the concentrate was applied to Sephadex G-25 column (commercially available from Pharmacia) preliminarily equilibrated with 10 mM potassium phosphate buffer (pH 7.8) with a volume of 5 times that of the column, thereby exchanging the buffer to 10 mM potassium phosphate buffer (pH 7.8). The resultant was passed through DEAE-Sepharose column (commercially available from Pharmacia) preliminarily equilibrated with 10 mM phosphate buffer (pH 7.8) with a volume of 5 times that of the column. The passed through fluid was concentrated by ultrafiltration

8

and the buffer was exchanged to 40 mM acetate buffer (pH 5.5) by gel permeation chromatography using Sephadex G-25 column (commercially available from Pharmacia). The resultant was applied to CM-Sepharose column (commercially available from Pharmacia) preliminarily equilibrated with 40 mM acetate buffer (pH 5.5) with a volume of 5 times that of the column so as to adsorb the proteins to the column. After washing the column with 40 mM acetate buffer (pH 5.5) with a volume of 3 times that of the column, the proteins were eluted by 180 mM acetate buffer (pH 5.5), and the fractions having SOD activity were recovered and combined. The thus obtained solution was subjected to electrophoresis. As a result, it was proved that the protein contained in the solution was a single protein having a total SOD activity of $2.1 \times 10^5$ units as shown in Table 3. Further, the specific activity of the protein was measured according to the method described in References 9 and 12, which was 3560 units/mg protein.

The manganese ion content of the thus obtained human Mn-SOD was 0.78 ion per one polypeptide chain, which was measured by atomic absorption spectrophotometry. The molecular weight of the thus isolated human Mn-SOD was measured, which was 48,000. Thus, it was proved that most of the thus isolated human Mn-SOD molecules were in the form of dimer.

## Table 3

| Sample | Amount of Total Protein (g/l medium) | Total SOD Activity (units/l medium) |
|---|---|---|
| Disrupted Cell Suspension | 1861 | $9.84 \times 10^5$ |
| After DEAE-Sepharose Column | 166 | $5.1 \times 10^5$ |
| After CM-Sepharose Column | 59 | $2.1 \times 10^5$ |

## REFERENCES

1. (Harris, J.I. and Steinman, H.M., Superoxide and Superoxide Dismutase, Michelson, A.M., McCord, J.M. and Friedovich, I. eds., Academic Press, London, pp.225-230 (1977)

2. Barra, D., Schinina, M.E., Simmaco, M., Bannister, J.V., Bannister, W.H., Rotilio, G. and Bossa, F., J. Biol. Chem. 259;12595-12601, (1984)

3. Beck, Y., Oren, R., Amit, B., Levanon, A., Gorecki, M. and Hartman, J.R., Nucleic Acid Res., 15;9076 (1987)

4. Heckl, K., Nucleic Acids Research, 16(13); 6224, (1988)

5. Ye-Shin Ho and James D. Crapo, FEBS LETTERS, 229(2), 256-260 (1988)

6. Japanese Laid Open Patent Application (Kokai) No. 317086/88

7. Maniatis, T. et al., Molecular Cloning, Glover, D.M. eds., Cold Spring Harbor Laboratory, (1982)

8. Caruthers, M.H. et al., Genetic Engineering, vol. 4, Setlow, J. and Hollaender, A. eds, Plenum Publishing Corp., New York, (1982)

9. McCord, J.M., Friedovich, 1., J. Biol. Chem. 244:6049-6055 (1969)

10. Shigenori TANAKA , Biotechnology Reagents, published by Kagaku Kogyo Nipposha, pp. 399-412, (1989)

11. (Baret, A., Jadot, G. and Michelson, A.M., Biochem. Phamacol., 33:2755-2760 (1984)

12. Lowry, O.H., Rosenbrough, N.J., Farr, A.L. and Randall, R.J., J. Biol. Chem., 193, 265, (1951)

13. Japanese Laid Open Patent Application (Kokai) No. 289187/87

14. Japanese Laid Open Patent Application (Kokai) No. 63383/89

15. Saiki, R.R., Scharf, S., Faloona, F., Mullis, K.B., Horn, G.T., Erlich, H.A. and Arnheim, N. (1985) Science, 230, 1350-1350

16. Yutaro Hayashi, Hideo Matsui and Toshio Takagi, Method in Enzymology, vol. 173, pp.514-528 (1989)

17. McCord, J.M., Boyle, J.A., Day,Jr., E.D., Rizzolo, L.J. and Salin, M.L., A Manganese-containing Superoxide Dismutase from Human Liver, pp. 129-138, In:Superoxide and Superoxide Dismutase, Michelson, A.M., McCord, J.M. and Fridvich, 1. (Eds) Academic Press, London (1977)

18. Beck, Y., Barfeld, D., Yavin, Z., Levanon, A., Gorecki, M. and Hartman, J.R., BIO/TECHNOLOGY, Vol. 6, 930-935, (1988)

19. Bachman, B., in "E. coli and S. typhimurium, cellular and molecular biology", Neidhart, P., ed., chart 14, vol. 2, p.1207

## Claims

1. A recombinant vector plasmid comprising:
   a ligated promoter including tac promoter and $P_L$ promoter;
   a structural gene encoding human manganese superoxide dismutase, which is located downstream said ligated promoter; and
   a terminator which acts in E. coli cell, which is located downstream said structural gene; said recombinant vector plasmid being capable of expressing said human manganese superoxide dismutase gene.

2. An E. coli transformant which is transformed with said recombinant vector plasmid of claim 1.

3. The E. coli transformant of claim 2, of which host cell does not produce E. coli manganese superoxide dismutase.

4. A process of producing human manganese superoxide dismutase comprising culturing said transformant of claim 2 in a condition to produce the human superoxide dismutase, and recovering the produced human superoxide dismutase.

5. The process of claim 4, wherein said culturing of said transformant is carried out in a culture medium containing 1 - 20 mM $Mn^{2+}$.

6. The process of claim 5, wherein the concentration of $Mn^{2+}$ is 10 - 15 mM.

7. A process of recovering human manganese superoxide dismutase from a solution containing soluble proteins in bacterial cells, comprising, in the order mentioned, the steps of:
   (a) passing said solution containing soluble proteins in said bacterial cells through an anion-exchange chromatography;
   (b) adjusting the pH of the passed through fluid from said anion-exchange chromatography to 6.0 - 6.7, and adjusting the salt concentration of said passed through fluid from said anion-exchange chromatography column to the degree capable of being adsorbed to a cation-exchange chromatography column used in the following step (c);
   (c) passing the resulting solution obtained in step (b) through a cation-exchange chromatography column;
   (d) passing through said cation-exchange chromatography column an elution buffer having a pH of 6.0 - 6.7 and a high salt concentration capable of eluting human manganese superoxide dismutase; and
   (e) recovering the eluted human superoxide dismutase.

8. The process of claim 7, wherein the pH of said passed through fluid is adjusted to 6.5 in step (b),

9. The process of claim 7, wherein the pH of said buffer used in step (d) is 6.5.

10. The process of claim 7, wherein the human manganese superoxide dismutase recovered by the process has not lees than 0.9 manganese ion per one human manganese superoxide dismutase polypeptide chain.

11. The process of claim 7, wherein the human manganese superoxide dismutase recovered by the process has a specific activity of not less than about 4200 units/mg.

12. The process of claim 7, wherein the human manganese superoxide dismutase recovered by the process has an average molecular weight of not less than 85000.

MetLysHisSerLeuProAspLeuProTyr AspTyrGlyAlaLeuGluProHisIleAsn AlaGlnIleMetGlnLeuHisHisSerLys

HisHisAlaAlaTyrValAsnAsnLeuAsn ValThrGluGluLysTyrGlnGluAlaLeu AlaLysGlyAspValThrAlaGlnIleAla

LeuGlnProAlaLeuLysPheAsnGlyGly GlyHisIleAsnHisSerIlePheTrpThr AsnLeuSerProAsnGlyGlyGlyGluPro

LysGlyGluLeuLeuGluAlaIleIleLysArg AspPheGlySerPheAspLysPheLysGlu LysLeuThrAlaAlaSerValGlyValGln

GlySerGlyTrpGlyTrpLeuGlyPheAsn LysGlnArgGlyHisLeuGlnIleAlaAla CysProAsnGlnAspProLeuGlnGlyThr

ThrGlyLeuIleProLeuLeuGlyIleAsp ValTrpGluHisAlaTyrTyrLeuGlnTyr LysAsnValArgProAspTyrLeuLysAla

IleTrpAsnValIleAsnTrpGluAsnVal ThrGluArgTyrMetAlaCysLysLys

F i g . 1

EP 0 462 836 A2

```
       10         20         30         40         50         60
CGATGAAGCA CAGCCTCCCC GACCTGCCCT ACGACTACGG CGCCCTGGAA CCTCACATCA
GCTACTTCGT GTCGGAGGGG CTGGACGGGA TGCTGATGCC GCGGGACCTT GGAGTGTAGT

       70         80         90        100        110        120
ACGCGCAGAT CATGCAGCTG CACCACAGCA AGCACCACGC GGCCTACGTG AACAACCTGA
TGCGCGTCTA GTACGTCGAC GTGGTGTCGT TCGTGGTGCG CCGGATGCAC TTGTTGGACT

      130        140        150        160        170        180
ACGTCACCGA GGAGAAGTAC CAGGAGGCGT TGGCCAAGGG AGATGTTACA GCCCAGACAG
TGCAGTGGCT CCTCTTCATG GTCCTCCGCA ACCGGTTCCC TCTACAATGT CGGGTCTGTC

      190        200        210        220        230        240
CTCTTCAGCC TGCACTGAAG TTCAATGGTG GTGGTCATAT CAATCATAGC ATTTTCTGGA
GAGAAGTCGG ACGTGACTTC AAGTTACCAC CACCAGTATA GTTAGTATCG TAAAAGACCT

      250        260        270        280        290        300
CAAACCTCAG CCCTAACGGT GGTGGAGAAC CCAAAGGGGA GTTGCTGGAA GCCATCAAAC
GTTTGGAGTC GGGATTGCCA CCACCTCTTG GGTTTCCCCT CAACGACCTT CGGTAGTTTG

      310        320        330        340        350        360
GTGACTTTGG TTCCTTTGAC AAGTTTAAGG AGAAGCTGAC GGCTGCATCT GTTGGTGTCC
CACTGAAACC AAGGAAACTG TTCAAATTCC TCTTCGACTG CCGACGTAGA CAACCACAGG

      370        380        390        400        410        420
AAGGCTCAGG TTGGGGTTGG CTTGGTTTCA ATAAGGAACG GGGACACTTA CAAATTGCTG
TTCCGAGTCC AACCCCAACC GAACCAAAGT TATTCCTTGC CCCTGTGAAT GTTTAACGAC

      430        440        450        460        470        480
CTTGTCCAAA TCAGGATCCA CTGCAAGGAA CAACAGGCCT TATTCCACTG CTGGGGATTG
GAACAGGTTT AGTCCTAGGT GACGTTCCTT GTTGTCCGGA ATAAGGTGAC GACCCCTAAC

      490        500        510        520        530        540
ATGTGTGGGA GCACGCTTAC TACCTTCAGT ATAAAAATGT CAGGCCTGAT TATCTAAAAG
TACACACCCT CGTGCGAATG ATGGAAGTCA TATTTTTACA GTCCGGACTA ATAGATTTTC

      550        560        570        580        590        600
CTATTTGGAA TGTAATCAAC TGGGAGAATG TAACTGAAAG ATACATGGCT TGCAAAAAGT
GATAAACCTT ACATTAGTTG ACCCTCTTAC ATTGACTTTC TATGTACCGA ACGTTTTTCA


AATGAAGCT
TTACTTCGA
```

Fig. 2

( A )

```
┌ F 1-1
CTAGATCGAT GAAGCACAGC CTCCCCGACC
    TAGCTA CTTCGTGTCG GAGGGGCTGG
  └


TGCCCTACGA CTACGGCGCC CTGGAACCTC
ACGGGATGCT GATGCCGCGG GACCTTGGAG
           F1-6 └┘
         └┌→ F1-2
ACATCAACGC GCAGATCATG CAGCTGCACC
TGTAGTTGCG CGTCTAGTAC GTCGACGTGG


ACAGCAAGCA CCACGCGGCC TACGTGAACA
TGTCGTTCGT GGTGCGCCGG ATGCACTTGT


                    └┌ F 1-3
ACCTGAACGT CACCGAGGAG AAGTACCAGG
TGGACTTGCA GTGGCTCCTC TTCATGGTCC
 └┘
F1-5

AGGCGTTGGC CAAGGGAGAT GTTACAGCCC
TCCGCAACCG GTTCCCTCTA CAATGTCGGG


          ┌
AGATAGCTCT TCAG
TCTATCGAGA AGTCTTAA
          F1-4 └┘
```

F i g . 3

( B )

↱ F2-1
AATTCTCTTG AGCCTGCACT GAAGTTCAAT
   GAGAAG TCGGACGTGA CTTCAAGTTA
   ↳

GGTGGTGGTC ATATCAATCA TAGCATTTTC
CCACCACCAG TATAGTTAGT ATCGTAAAAG
   F2-8 ↵↳
        ↰↱ F2-2
TGGACAAACC TCAGCCCTAA CGGTGGTGGA
ACCTGTTTGG AGTCGGGATT GCCACCACCT


GAACCCAAAG GGGAGTTGCT GGAAGCCATC
CTTGGGTTTC CCCTCAACGA CCTTCGGTAG
                        F2-7 ↵↳

AAACGTGACT TTGGTTCCTT TGACAAGTTT
TTTGCACTGA AACCAAGGAA ACTGTTCAAA

↰↱ F2-3
AAGGAGAAGC TGACGGCTGC ATCTGTTGGT
TTCCTCTTCG ACTGCCGACG TAGACAACCA


GTCCAAGGCT CAGGTTGGGG TTGGCTTGGT
CAGGTTCCGA GTCCAACCCC AACCGAACCA
   F2-6 ↵↳
              ↰↱ F2-4
TTCAATAAGG AACGGGGACA CTTACAAATT
AAGTTATTCC TTGCCCCTGT GAATGTTTAA

                  ↰
GCTGCTTGTC CAAATCAG
CGACGAACAG GTTTAGTCCT AG
                        ↵
                    F2-5

Fig. 3 ( Continued )

( C )

F3-1

GATCCACTGC AAGGAACAAC AGGCCTTATT
    GTGACG TTCCTTGTTG TCCGGAATAA

CCACTGCTGG GGATTGATGT GTGGGAGCAC
GGTGACGACC CCTAACTACA CACCCTCGTG
  F3-6
    F3-2
GCTTACTACC TTCAGTATAA AAATGTCAGG
CGAATGATGG AAGTCATATT TTTACAGTCC

CCTGATTATC TAAAAGCTAT TTGGAATGTA
GGACTAATAG ATTTTCGATA AACCTTACAT
     F3-5
     F3-3
ATCAACTGGG AGAATGTAAC TGAAAGATAC
TAGTTGACCC TCTTACATTG ACTTTCTATG

ATGGCTTGCA AAAAGTAATG A
TACCGAACGT TTTTCATTAC TTCGA
       F3-4

Fig. 3 ( Continued )

block A          block B          block C

— — —        — — —        — — — ÷ —
 — — —        — — —        — — — —

↓←T4ligase      ↓←T4ligase       ↓←T4ligase

═════════      ═════════       ═════════

↓←PUC19        ↓←PUC19         ↓←PUC19
  /Xbal-EcoRI     /EcoRl-BamHI      /BamHI-HindIII
↓←T4ligase      ↓←T4ligase       ↓←T4ligase

Xbal  EcoRI      EcoRl  BamHl      BamHl  HindⅢ
  SODA            SODB            SODC

pUCSODA         pUCSODB         pUCSODC

  Ampr  Scal      Ampr           Ampr
              Scal              Scal

↓←/EcoRl-EarI    ↓←/EarI-BamHI    ↓←/BamHl-HindIII
↓←Isolate 3kbp   ↓←Isolate CA260bp ↓←Isolate CA180bp
↓              ↓             SOD/Block C
───────────────────────

↓←T4ligase

Xbal
  SODA+B  BamHl

pUCSOD(A+B)

  Ampr
        Scal

PSW115
 ↓←/Clal-HindIII
 ↓←Isolate CA3.6kbp
 ↓←T4ligase +(SOD/Block A+B)
    +(SOD/Block C)
 ↓
pSWTPSOD

↓←/ClaI-BamHI
↓←Isolate CA430bp
 SOD/Block·A+B

              Clal
          Tac Pc Human Mn SOD

        pSWTPSOD
                      HindⅢ

          Ampr
                Scal

F i g .  4

1 2 3 4 5 6 7 8 9

# FIG. 5

p S W T P S O D

↓

↓ ←— HindIII Digestion

↓ ←— Isolate CA 4.9kbp

↓ ←— Hind III Digestion

↓ ←— Klenow Treatment

↓ ←— T4Ligase

↓

p P$_L$ S O D

Fig. 6

MetLysHisSerLeuProAspLeuProTyr AspTyrGlyAlaLeuGluProHisIleAsn AlaGlnIleMetGlnLeuHisHisSerLys

HisHisAlaAlaTyrValAsnAsnLeuAsn ValThrGluGluLysTyrGlnGluAlaLeu AlaLysGlyAspValThrAlaGlnThrAla

LeuGlnProAlaLeuLysPheAsnGlyGly GlyHisIleAsnHisSerIlePheTrpThr AsnLeuSerProAsnGlyGlyGlyGluPro

LysGlyGluLeuLeuGluAlaIleLysArg AspPheGlySerPheAspLysPheLysGlu LysLeuThrAlaAlaSerValGlyValGln

GlySerGlyTrpGlyTrpLeuGlyPheAsn LysGlnArgGlyHisLeuGlnIleAlaAla CysProAsnGlnAspProLeuGlnGlyThr

ThrGlyLeuIleProLeuLeuGlyIleAsp ValTrpGluHisAlaTyrTyrLeuGlnTyr LysAsnValArgProAspTyrLeuLysAla

IleTrpAsnValIleAsnTrpGluAsnVal ThrGluArgTyrMetAlaCysLysLys

Fig. 7

```
              10          20          30          40          50          60
      CGATGAAGCA  CAGCCTCCCC  GACCTGCCCT  ACGACTACGG  CGCCCTGGAA  CCTCACATCA
      GCTACTTCGT  GTCGGAGGGG  CTGGACGGGA  TGCTGATGCC  GCGGGACCTT  GGAGTGTAGT

              70          80          90         100         110         120
      ACGCGCAGAT  CATGCAGCTG  CACCACAGCA  AGCACCACGC  GGCCTACGTG  AACAACCTGA
      TGCGCGTCTA  GTACGTCGAC  .GTGGTGTCGT  TCGTGGTGCG  CCGGATGCAC  TTGTTGGACT

             130         140         150         160         170         180
      ACGTCACCGA  GGAGAAGTAC  CAGGAGGCGT  TGGCCAAGGG  AGATGTTACA  GCCCAGACAG
      TGCAGTGGCT  CCTCTTCATG  GTCCTCCGCA  ACCGGTTCCC  TCTACAATGT·CGGGTCTGTC

             190         200         210         220         230         240
      CTCTTCAGCC  TGCACTGAAG  TTCAATGGTG  GTGGTCATAT  CAATCATAGC  ATTTTCTGGA
      GAGAAGTCGG  ACGTGACTTC  AAGTTACCAC  CACCACTATA  CTTACTATCC  TAAAACACCT

             250         260         270         280         290         300
      CAAACCTCAG  CCCTAACGGT  GGTGGAGAAC  CCAAAGGGGA  GTTGCTGGAA  GCCATCAAAC
      GTTTGGAGTC  GGGATTGCCA  CCACCTCTTG  GGTTTCCCCT  CAACGACCTT  CGGTAGTTTG

             310         320         330         340         350         360
      GTGACTTTGG  TTCCTTTGAC  AAGTTTAAGG  AGAAGCTGAC  GGCTGCATCT  GTTGGTGTCC
      CACTGAAACC  AAGGAAACTG  TTCAAATTCC  TCTTCGACTG  CCGACGTAGA  CAACCACAGG

             370         380         390         400         410         420
      AAGGCTCAGG  TTGGGGGTTGG  CTTGGTTTCA  ATAAGGAACG  GGGACACTTA  CAAATTGCTG
      TTCCGAGTCC  AACCCCAACC  GAACCAAAGT  TATTCCTTGC  CCCTGTGAAT·GTTTAACGAC

             430         440         450         460         470         480
      CTTGTCCAAA  TCAGGATCCA  CTGCAAGGAA  CAACAGGCCT  TATTCCACTG  CTGGGGATTG
      GAACAGGTTT  AGTCCTAGGT  GACGTTCCTT  GTTGTCCGGA  ATAAGGTGAC  GACCCCTAAC

             490         500         510         520         530         540
      ATGTGTGGGA  GCACGCTTAC  TACCTTCAGT  ATAAAAATGT  CAGGCCTGAT  TATCTAAAAG
      TACACACCCT  CGTGCGAATG  ATGGAAGTCA  TATTTTTACA  GTCCGGACTA  ATAGATTTTC

             550         560         570         580         590         600
      CTATTTGGAA  TGTAATCAAC  TGGGAGAATG  TAACTGAAAG  ATACATGGCT  TGCAAAAAGT
      GATAAACCTT  ACATTAGTTG  ACCCTCTTAC  ATTGACTTTC  TATGTACCGA  ACGTTTTTCA


      AATGAAGCT
      TTACTTCGA
```

Fig. 8

Fig. 9